# EUROPEAN PATENT SPECIFICATION

(11) **EP 3 930 710 B1**
(45) Date of publication and mention of the grant of the patent: **14.10.2026**
(21) Application number: 20713372.9
(22) Date of filing: 02.03.2020
(51) Int. Cl.: A61K 31/409, A61P 3/04, A23L 33/00, A61K 36/00, C07D 487/22, A23L 33/10, C12N 1/205, C12P 17/18

(54) **HYDROXYPHEOPHORBIDE COMPOUNDS, METHODS AND USES THEREOF**
HYDROXYPHEOPHORBIDVERBINDUNGEN, VERFAHREN UND VERWENDUNGEN DAVON
COMPOSÉS D'HYDROXYPHÉOPHORBIDE, PROCÉDÉS ET LEURS UTILISATIONS

(30) Priority: 01.03.2019 PT 2019115348
(43) Date of publication of application: 05.01.2022
(73) Proprietor: CIIMAR - Centro Interdisciplinar de Investigação Marinha e Ambiental, 4450-208 Matosinhos (PT); Universidade Do Porto, 4099-002 Porto (PT)
(72) Inventor: URBATZKA, Ralph, 4450-208 Matosinhos (PT); FREITAS, Sara, 4450-208 Matosinhos (PT); COSTA LEÃO, Pedro Nuno, 4450-208 Matosinhos (PT); SOUSA, Maria Ligia, 4450-208 Matosinhos (PT); ALVES REIS, Mariana, 4450-208 Matosinhos (PT); GONÇALVES SILVA, Natália, 4450-208 Matosinhos (PT); DE OLIVEIRA E VASCONCELOS, Vitor Manuel, 4450-208 Matosinhos (PT); RIBEIRO, Tiago, 4450-208 Matosinhos (PT); ROSA, Filipa, 4450-208 Matosinhos (PT)
(74) Representative: Patentree
(86) International application number: PCT/IB2020/051759
(87) International publication number: WO 2020/178713

(56) References cited:
- US-A1- 2011 009 616
- SAKDARAT S ET AL: "Bioactive constituents from the leaves of Clinacanthus nutans Lindau", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 17, no. 5, 1 March 2009 (2009-03-01), pages 1857 - 1860, XP025992055, ISSN: 0968-0896, [retrieved on 20090131], DOI: 10.1016/J.BMC.2009.01.059
- ALINE KELY FELÍCIO DE SOUSA SANTOS ET AL: "Antinociceptive activity of Sargassum polyceratium and the isolation of its chemical components", REVISTA BRASILEIRA DE FARMACOGNOSIA- BRAZILIAN JOURNAL OF PHARMACOGNOSY, vol. 25, no. 6, 1 November 2015 (2015-11-01), BR, pages 683 - 689, XP055696313, ISSN: 0102-695X, DOI: 10.1016/j.bjp.2015.07.017
- NAOYUKI ISHIDA ED - CHANKVETADZE B ET AL: "Expanded separation technique for chlorophyll metabolites in Oriental tobacco leaf using non aqueous reversed phase chromatography", JOURNAL OF CHROMATOGRAPHY A, ELSEVIER, AMSTERDAM, NL, vol. 1218, no. 34, 22 June 2011 (2011-06-22), pages 5810 - 5818, XP028254672, ISSN: 0021-9673, [retrieved on 20110630], DOI: 10.1016/J.CHROMA.2011.06.082
- AMIRA ARCINIEGAS ET AL: "Anti-hyperglycemic, antioxidant, and anti-inflammatory activities of extracts and metabolites from Sida acuta and Sida rhombifolia", QUIMICA NOVA, 27 October 2016 (2016-10-27), BR, XP055696309, ISSN: 0100-4042, DOI: 10.21577/0100-4042.20160182
- SEMAAN D G ET AL: "In vitroanti-diabetic effect of flavonoids and pheophytins fromAllophylus cominia Sw. on the glucose uptake assays by HepG2, L6, 3T3-L1 and fat accumulation in 3T3-L1 adipocytes", JOURNAL OF ETHNOPHARMACOLOGY, ELSEVIER IRELAND LTD, IE, vol. 216, 12 January 2018 (2018-01-12), pages 8 - 17, XP085357531, ISSN: 0378-8741, DOI: 10.1016/J.JEP.2018.01.014
- SARA FREITAS ET AL: "Chlorophyll Derivatives from Marine Cyanobacteria with Lipid-Reducing Activities", MARINE DRUGS, vol. 17, no. 4, 17 April 2019 (2019-04-17), pages 229, XP055696256, DOI: 10.3390/md17040229

## Description

### TECHNICAL FIELD

The present disclosure relates to 13²-hydroxypheophorbide compounds for use in the therapy or treatment of metabolic diseases.

### BACKGROUND

Obesity is an increasing epidemic, since a higher percentage of the world's population is overweight, and is associated with several chronic diseases like diabetes, cardiovascular diseases and cancer. Some natural products are being used in the clinic as anti-obesogenic compounds, as for example Orlistat, a synthetic derivative of lipostatin isolated from *Streptomyces toxytricini.*

Chlorophyll molecules and their main derivatives (pheophytins, pheophorbides) are known for a long time. Some beneficial bioactivities were described for them including (1) antioxidant properties (Kang et al., 2018), (2) anti-inflammatory activity (Kang et al., 2018; Subramoniam et al., 2012), (3) anti-mutagenic activity (Ferruzzi et al., 2002), (4) cytotoxic effects on cancer cells (Zhao et al., 2014), (5) antiviral activity against hepatitis C virus (Wang et al., 2009), (6) antimicrobial activity (Gomes et al., 2015) and (7) induction of neuro-differentiation (Ina et al., 2007). Reduction of lipid content in murine 3T3L1 cells was shown for a pheophytin rich extract of a plant at 100 µg/ml (Seemann et al., 2018). However, for 13²-(*S*)-hydroxy-pheophytin a *(hpa)* only few bioactivities are known. *Hpa* was shown to have a low cytotoxicity on cancer cells (8.8 - 22 µg/ml, Cheng et al., 2001,). A methanolic extract from red algae containing *hpa* amongst 7 other compounds (major peaks by unidentified compounds) showed anti-inflammatory activity *in vitro* at 10 µg/ml (Shu et al., 2013). Until this moment, no utility was demonstrated for *hpa* in relation to obesity, or obesity-related co-morbidities as diabetes or fatty liver disease or any effects of lipid metabolic diseases..

These facts are disclosed in order to illustrate the technical problem addressed by the present disclosure.

**GENERAL DESCRIPTION** The references to methods of treatment in the summary and detailed description are to be interpreted as references to the compounds, pharmaceutical compositions and medicaments of the present invention **for use** in a method for treatment of the human (or animal) body by therapy (or for diagnosis).

The present invention is defined by the claims.

The hydroxypheophorbide compounds can be used for reduction of lipids, and possibly for the treatment of obesity or related diseases. They have the advantage that they are from a natural resource and can be marketed as natural products.

In the present disclosure a known and an unprecedented hydroxypheophorbide were isolated from marine cyanobacteria: 13²-(*S*)-hydroxy-pheophytin a (hpa), and the 13²-(*S*)-hydroxy-pheofarnesin a (hfa), respectively. Hpa and hfa demonstrated significant lipid reducing activities with an IC₅₀ of 8.9 ± 0.4 and 15.5 ± 1.3 µM, respectively, in the zebrafish Nile red fat metabolism assay. Bioactivity was further confirmed in differentiated murine pre-adipocytes grown as 3D spheroids. Since these molecules are shown to be produced from organisms that satisfy the GRAS criteria (generally regarded as safe), their lipid reducing activities can be marketed as novel natural products in the form of nutraceuticals or food supplements for the treatment of obesity or related co-morbidities. Commercialization as isolated molecules or as raw material would be also possible.

The hydroxypheophorbide compounds of the presented disclosure were isolated by bio-assay guided fractionation approaches based on the zebrafish Nile red fat metabolism assay. 13²-(*S*)-Hydroxy-pheofarnesin a *(hfa)* is a novel compound, reported for the first time. Moreover, it is disclosed for the first time that *hpa* and *hfa* have lipid reducing activities in two different model systems, in the zebrafish Nile red fat metabolism assay and in differentiated murine pre-adipocytes grown as 3D spheroids

An aspect of the present disclosure relates to hydroxypheophorbide compounds with bioactivities towards obesity and obesity-related co-morbidities.

Therefore, the present subject-matter discloses compounds or a pharmaceutically acceptable salt, hydrate, solvate, N-oxide, stereoisomer, diastereoisomer, enantiomer or atropisomer, polymorph for use in the therapy or treatment of metabolic diseases comprising the following formula 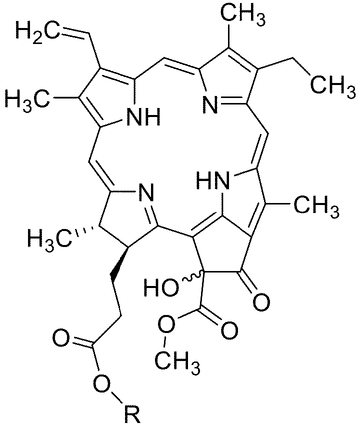 wherein R is CₙH(₂ₙ₋₁) and n is an entire number multiple of 5.

In an embodiment, n may be selected from 5 or 10 or 15 or 20 or 25 or 30, preferably 15 or 20.

In an embodiment, R may be C₅H₉, C₁₀H₁₉ or C₁₅H₂₉ or C₂₀H₃₉ or C₂₅H₄₉ or C₃₀H₅₉, preferably C₁₅H₂₉ or C₂₀H₃₉.

In an embodiment, R may be 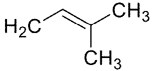 or 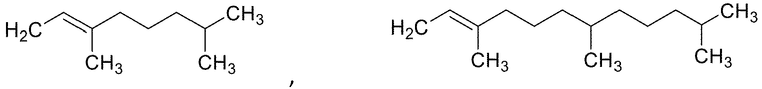 or 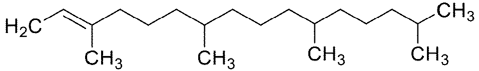 or 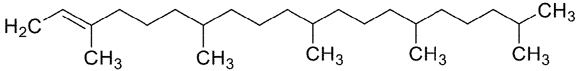 or 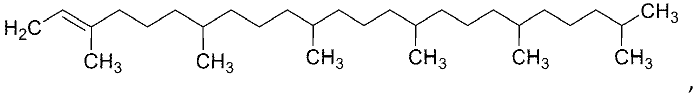 preferably 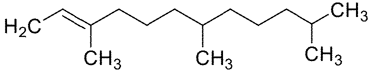 or

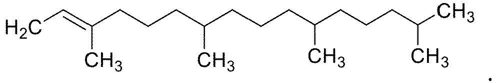

In an embodiment, the compound of the present disclosure may be use in the therapy or treatment of metabolic diseases. Preferably in therapy or treatment of obesity, an obesity-related disorder, an obesity related disease, overweight, an obesity-related condition, or lipid obesity disorders. More preferably, morbid obesity, visceral fat obesity, diabetes, insulin-resistance syndrome, nonalcoholic steatohepatitis, a cardiovascular disorder, renal disorders, pancreatic disorders, polycystic ovary syndrome, metabolic syndrome or fatty liver.

In an embodiment for better results, the compound is: 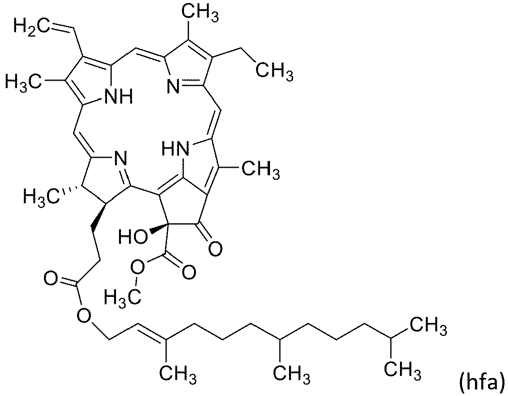 or;

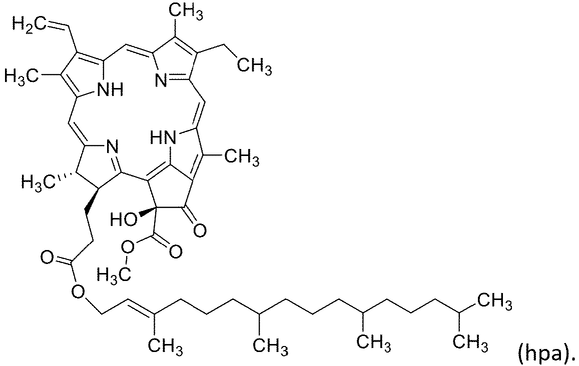

Another aspect of the present disclosure is a composition comprising at least a compound, pharmaceutically acceptable salt, hydrate, solvate, N-oxide, stereoisomer, diastereoisomer, enantiomer, atropisomer, or polymorph thereof comprising the following formula: 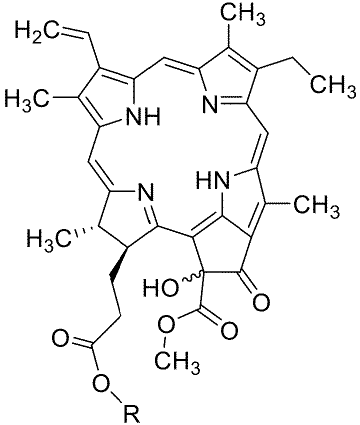
wherein R is CₙH(₂ₙ₋₁)
n is an entire number multiple of 5 and
n is different from 20. Preferably, wherein n is selected from 5 or 10 or 15 or 25 or 30, more preferably n is 15.

In an embodiment, R may be C₅H₉, C₁₀H₁₉ or C₁₅H₂₉ or C₂₅H₄₉ or C₃₀H₅₉, preferably C₁₅H₂₉

In an embodiment, R may be 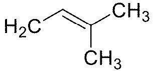 or 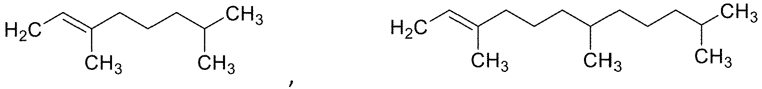 or 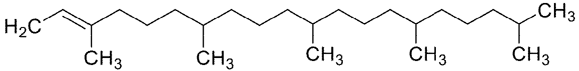 or 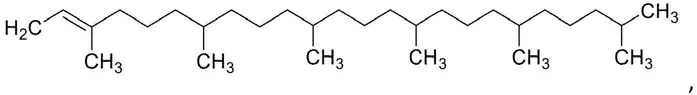 preferably

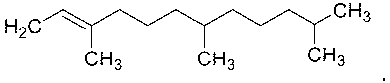

Another aspect of the present disclosure relates to a composition for use in the therapy or treatment of metabolic diseases comprising at least a compound, pharmaceutically acceptable salt, hydrate, solvate, N-oxide, stereoisomer, diastereoisomer, enantiomer, atropisomer, or polymorph thereof, as defined in any of the previous claims and a pharmaceutically acceptable carrier, adjuvant, excipient, emulsification agent, or mixtures thereof.

In an embodiment, the composition is a pharmaceutical composition, a foodstuff composition, a dietary composition, a nutraceutical composition or an anti-obesity composition.

Another aspect of the present disclosure relates to a foodstuff or a nutraceutical comprising the compound of claims 7-10 or composition comprising the compound of claims 7-10.

Another aspect of the present disclosure relates to the use of a hydroxypheophorbide compound or composition as an anti-obesity agent.

Another aspect of the present disclosure relates to a method of treating or preventing obesity or related diseases comprising administering a therapeutically effective amount of a compound or a composition as defined in any of the previous claims.

Another aspect of the present disclosure relates to a process for the production of the compound of formula 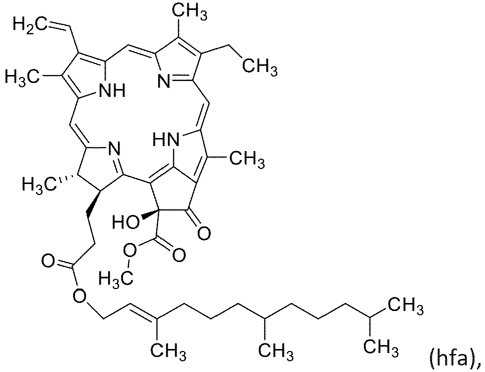 and its isolation from an extract of *Nodosilinea sp.* LEGE06001.

Another aspect of the present disclosure relates to the compound comprises the formula 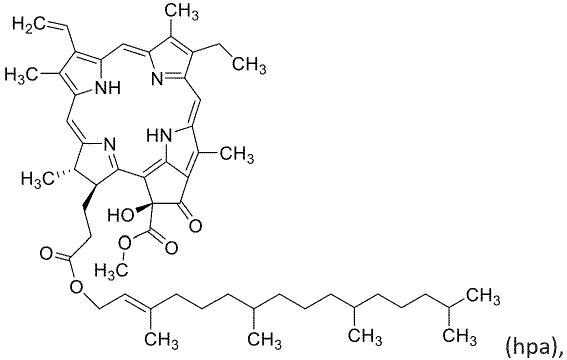 and its isolation from an extract of *Cyanobium* sp. LEGE 07175.

### BRIEF DESCRIPTION OF THE DRAWINGS

The following figures provide preferred embodiments for illustrating the disclosure and should not be seen as limiting the scope of invention.

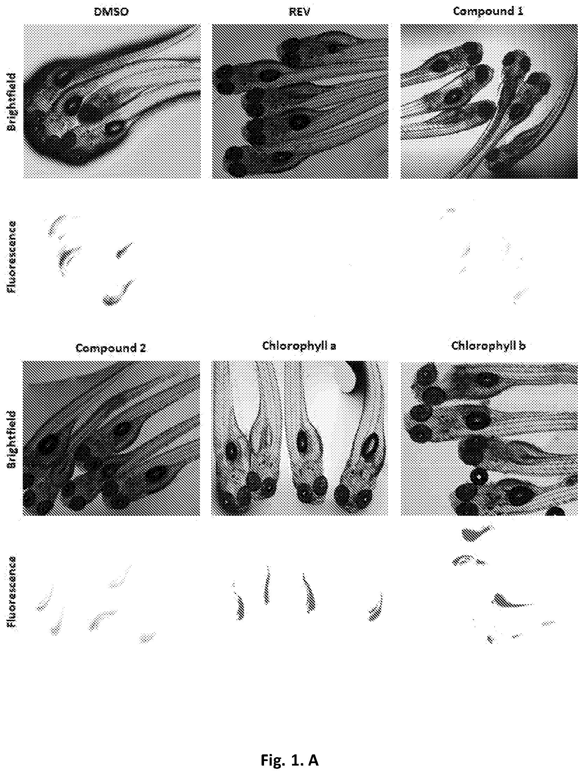

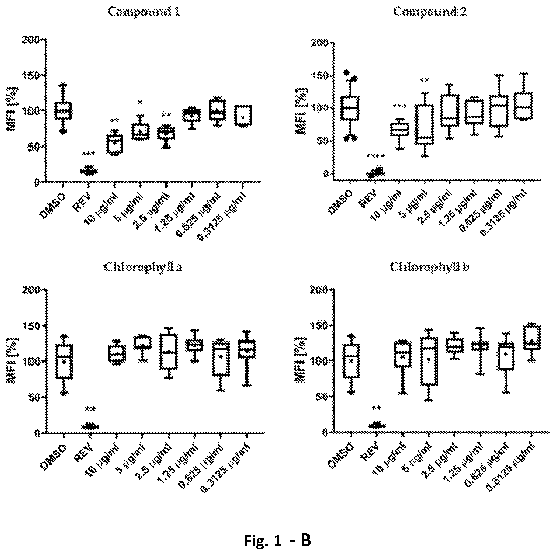

**Figure 1****.A:** Representative images of the zebrafish Nile red fat metabolism assay. Nile red fluorescence signal indicates neutral lipids around the yolk sac and stomach/intestine. Compound 1 (hpa) and compound 2 (hfa) show a decrease in the Nile red staining that is not present when zebrafish were exposed to chlorophylls a and b. A solvent control (0.1% dimethyl sulfoxide, DMSO) and positive control group (50 µM resveratrol, REV) was included.
**Figure 1****.B:** Quantification of lipid-reducing activity in the zebrafish Nile Red fat metabolism assay after exposure for 48h. Values are expressed as mean fluorescence intensity (MFI) relative to the DMSO group and are derived from 6 to 8 individual larvae per treatment group. The data are represented as box-whisker plots (5 to 95 percentiles). Asterisks highlight significant differences compared to the DMSO group (**** p < 0.0001; *** p < 0.001; ** p < 0.01; * p < 0.05).

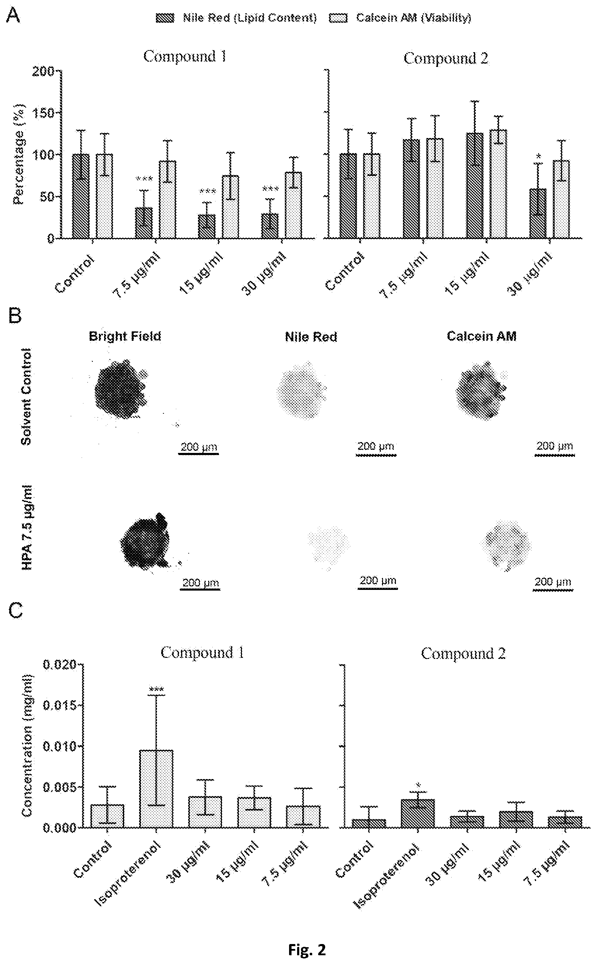

**Figure 2****:** Evaluation of lipid content (Nile red) and viability (calcein AM) in differentiated 3T3-L1 spheroids after exposure to compound 1 (hpa) and compound 2 (hfa) over 48h. Panel A shows the results of quantification of fluorescence by CellProfiler software, and Panel B some representative images from fluorescence microscopy (both fluorescence channels were converted into black and white for better visualization). Statistical difference to the solvent control were analysed by One-Way ANOVA, followed by a Dunnett's multiple comparison post-test (*** p- value <0.001, ** p-value < 0.01, * p-value < 0.05). Quantification of free glycerol (lipolysis) on the medium where 3T3-L1 organoids were exposed to compound 1 (hpa) and compound 2 (hfa) over 48h. No significant alterations on free glycerol content in the medium were observed. Kolmogorov-Smirnov test was used to test normality of the data, followed by a Dunnett's multiple comparison post-test (*** p- value <0.001, ** p-value < 0.01, * p-value < 0.05).

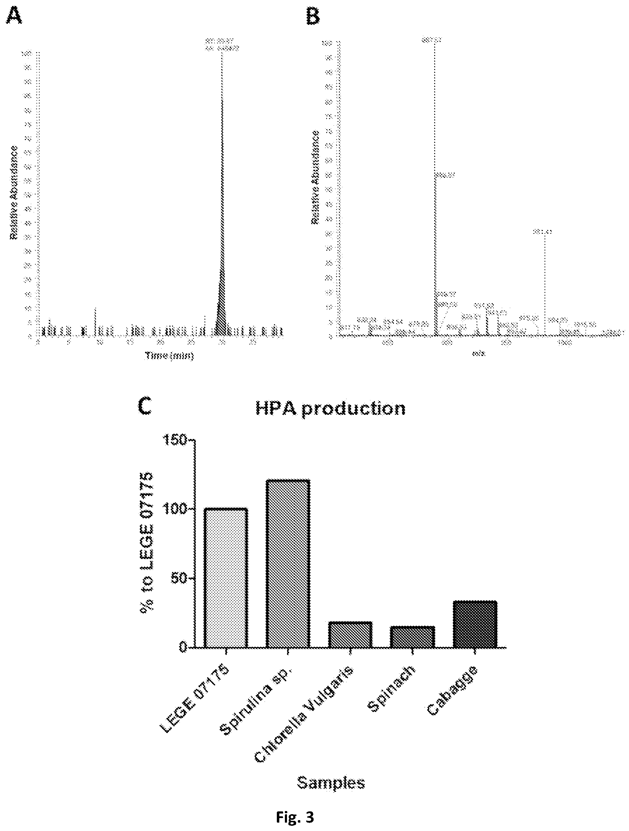

**Figure 3****:** Comparative quantification of hpa from different sources. A Total Ion Chromatogram (TIC) from hpa standard at a concentration of 16 µg/mL in MeOH (100%), in negative mode. The standard shows a single peak with a retention time of 29.67 minutes and the pattern of fragmentation is shown on panel B (887.57 - 888.57 - 889.57 - 890.58 m/z). C) Quantification data of hpa are shown as percentage relative to LEGE 07175 (area of the peaks on the TIC on the selected mass range (887.50-888.50 m/z) with absorption at 428 nm on PDA).

### DETAILED DESCRIPTION

The present disclosure relates to hydroxypheophorbide compounds, in particular 13²-hydroxy-pheophorbide compounds, for use in the therapy or treatment of metabolic diseases

Therefore, the present subject-matter discloses compounds or a pharmaceutically acceptable salt, hydrate, solvate, N-oxide, stereoisomer, diastereoisomer, enantiomer or atropisomer, polymorph for use in medicine comprising the following formula 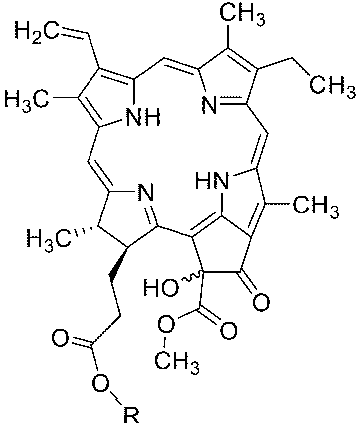 wherein R is CₙH(₂ₙ₋₁) and n is an entire number multiple of 5. Preferably, n may be selected from 5 or 10 or 15 or 20 or 25 or 30, preferably 15 or 20.
The compounds of the present disclosure may be use in the therapy or treatment of obesity, an obesity-related disorder, an obesity related disease, being overweight, an obesity-related condition, or lipid obesity disorders.

### Structure of 13²(S)-hydroxy-pheofarnesin a (hfa)

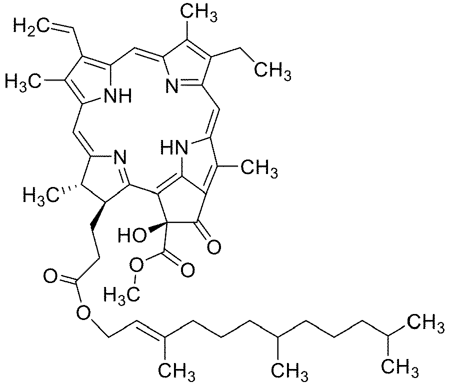

### Structure of 13²(R)-hydroxy-pheofarnesin a

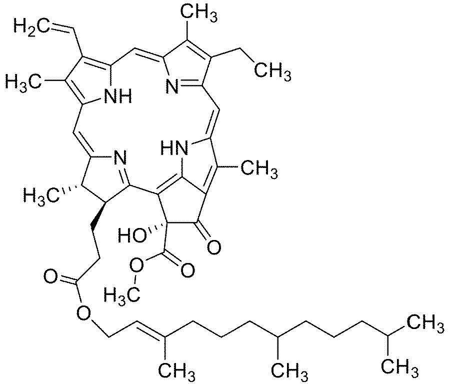

### Structure of 13²(S)-hydroxy-pheophytin a (hpa)

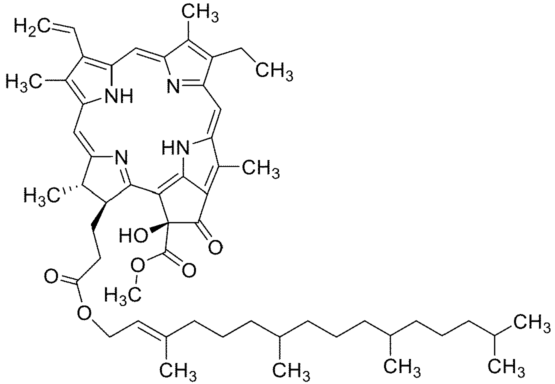

### Structure of 13²(R)-hydroxy-pheophytin a

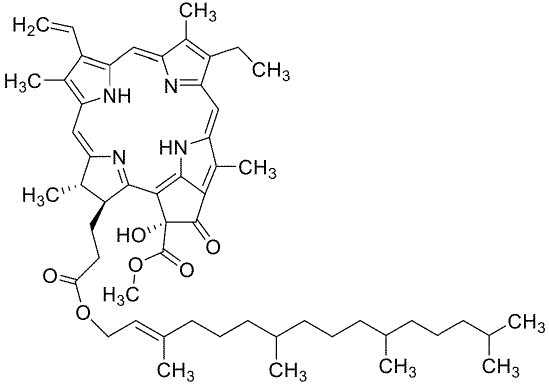

This disclosure uncovers compounds with novel lipid reducing activity. It is reported the isolation procedure of two hydroxypheophorbide compounds (hpa and hfa), and the structural elucidation of hfa. Hpa was isolated from *Cyanobium sp.* LEGE07175 and hfa from *Nodosilinea sp.* LEGE06001. These marine cyanobacteria are commercially available and can be purchased at http://lege.ciimar.up.pt/ordering-services/). Their lipid reducing activity was evaluated using the zebrafish Nile red fat metabolism assay and a 3D cell culture model of murine pre-adipocytes. Furthermore, the production of hpa was evaluated in different materials (Spirulina, microalgae, spinach, cabbage), with GRAS status (generally regarded as safe) for human consumption, to foster the commercialization of the compounds as novel natural products in the form of nutraceuticals or food supplements for the treatment of obesity or related co-morbidities.

In an embodiment, cyanobacterial growth, extraction and fractionation were performed. Cyanobacteria strains *Cyanobium sp.* LEGE07175 and *Nodosilinea sp.* LEGE06001 were isolated from the Portuguese coast and are maintained in the LEGEcc in CIIMAR, Matosinhos, Portugal (the strains are commercial and can be purchased at http://lege.ciimar.up.pt/ordering-services/). The strains were cultured in Z8 medium supplemented with marine tropical salt (25 g/L), at 25°C, with a photoperiod of 14h/10h light and dark respectively, and at light intensity of 10 µmols photons s1m2. *Nodosilinea sp.* LEGE 06001 and *Cyanobium sp.* LEGE 07175 cultures were grown in 20 L flasks with constant aeration and at the exponential phase, cells were harvested through centrifugation, frozen and freeze-dried. The biomass of LEGE 07175 (13 g) and LEGE 06001 (56.5 g) was extracted by repeated percolation with warm mixture of CH2Cl2/MeOH (2:1, v/v) yielding a crude extract of 1.9 g and 8.74 g, respectively. Both crude extracts were fractionated by normal-phase (Si gel 60, 0.015-0.040 mm, Merck KGaA) VLC with an increasing polarity grade, from 90% n-hex to 100% EtOAc and 100% MeOH, giving a total of 9 fractions each.

In an embodiment, hpa was obtained as a green dark amorphous solid, after several chromatographic steps of an extract from the cyanobacterium *Cyanobium sp.* LEGE 07175 (the strain is commercially available at http://lege.ciimar.up.pt/ordering-services/). The isolation was guided by a strong the reduction of lipid content observed in the zebrafish Nile red fat metabolism assay. The structural assignment of hpa was obtained through comparison of the obtained spectroscopic data with those reported on the literature. The HR-ESI-MS analysis showed a monoisotopic m/z 887.5697 [M + H]⁺, consistent with the molecular formula of C₅₅H₇₄N₄O₆. The 1D and 2D NMR experiments analysis revealed the typical resonances and correlations for this compound. The presence of the alcohol group and other functional groups were corroborated using NMR and HR-ESI-MS/MS.

The bioassay guided fractionation of the extract of *Nodosilinea sp* LEGE 06001 (strain commercially available at http://lege.ciimar.up.pt/ordering-services/) yielded hfa as a brownish green amorphous solid. The molecular formula was assigned as C₅₀H₆₄N₄O₆ on the basis of HR-ESI-MS data, *m*/*z* 817.4505 [M + H]⁺, which demanded fifteen degrees of unsaturation.

The ¹H and ¹³C NMR data of hfa showed similarities to what was observed for **hpa,** indicating **hfa** to be a hydroxypheophorbide compound. Through the comparison of the HR-ESI-MS data it could be concluded that compound **hfa** bears a farnesyl moiety instead of a phytyl. The difference between the mass of the pseudomolecular ion at *m*/*z* 817.4505 [M + H] ⁺ and the fragment at *m*/*z* 609.2702 [M - farnesyl]⁺ (Δ *m*/*z* 208.1803) is consistent with the presence of a farnesyl substituent in opposition to phytyl (Δ *m*/*z* 279.36; *m*/*z* 909.55 [M + Na]⁺ and *m*/*z* 607.20 [M - phytol]⁺. Therefore, compound hfa was named hydroxyl-pheofarnesine a.

Hence, the H NMR spectrum, showed all the typical resonances of the porphyrin ring: three singlets and one multiplet aromatic methyl groups (δ_{H} 1.59 m, 3.28 s, 3.44 s, 3.90 s), one aromatic ethyl group (δ_{H} 1.72, 3.64, 3.77), three olefinic singlets (δ 8.71, 9.56, 9.77), a methoxyl group (δ 3.76) and a vinyl substitution (δ_{H} 6.19, 6.35, 8.03) with a characteristic exomethylene coupling pattern (*J* = 18.7 and J = 11.4 Hz). The attached farnesyl moiety was recognized by the carbinol resonances (δ_{H} 4.44, 4.5 at F1), the vinylic resonances for H-F2 (δ_{H} 5.14) and H₃-F3¹ (δ_{H} 1.61), and the germinal methyl's at F11¹ and F12 (δ_{H} 0.85 d).

Zebrafish *(Danio rerio)* has become an attractive model organism for biomedical research. In the study of complex metabolic disorders like obesity, the use of more complex *in vivo* model systems as small whole animal models can bring significant advantages. For drug discovery, zebrafish assays can be used complementary to rodent assays, with easier handling, high predictive validity and cost-efficiency while compatible with high-throughput screening. Zebrafish possess higher physiological relevance than cellular *in vitro* models and have genetic homology to mammals, including significant similarities in lipid metabolism. The zebrafish Nile red fat metabolism assay analyses the capacity of compounds to reduce neutral lipids in zebrafish larvae in vivo.

13²(S)-hydroxy-pheophytin a *(hpa)* is a known compound, and belongs to the photosystem II complex of the chloroplast. The second is a novel compound, 13²(S)-hydroxy-pheofarnesin a *(hfa),* which is structurally related to *hpa.* Both compounds *(hpa, hfa)* have a lipid reducing activity in the zebrafish Nile red fat metabolism assay (Urbatzka et al., 2018) with IC₅₀ values of 8.9 ± 0.4 and 15.5 ± 1.3 µM, respectively. No general toxicity was observed for both compounds in the same concentration ranges on zebrafish larvae (death, malformations). In contrast, exposure of zebrafish larvae to chlorophyll a and chlorophyll b did not demonstrate any lipid reducing activity.

In an embodiment, the bioactivity of *hpa* and hfa was confirmed in a further assay, using the murine pre-adipocyte cell line 3T3L1. Cells grown and differentiated as spheroids were exposed to *hpa* and hfa, respectively, and reduced significantly the lipid content of the spheroids. No toxicity was observed by calcein AM staining, in contrast non-specific cellular esterase activity was increased. Lipolysis was analyzed as potential mechanism, but *hpa* or hfa exposure of spheroids did not alter lipolytic activities. In an embodiment, the activity in differentiated 3T3L1 spheroids was confirmed and analysis of lipolysis performed. 3T3-L1 spheroids exposed after 7d differentiation, illustrated in Figure 2, showed significant reduction of lipid accumulation after 48h of exposure to **hpa** at 7.5, 15 and 30 µg/mL, and to **hfa** at 30 µg/mL. Exposure to 15 and 30 µg/mL of **hpa** reduced the viability of spheroids for 20%, although not significant, while exposure to **hfa** did not cause any reduction in viability at any concentration.

In an embodiment, chlorophyll a and chlorophyll b were tested for lipid reducing activity on the zebrafish Nile red fat metabolism assay. These compounds did not reduce the fluorescence intensity of Nile red in the assay at all tested concentrations (156 ng/mL to 10 µg/mL) as demonstrated in figure 1(B), highlighting the specificity of the disclosed hydroxypheophorbide compounds for lipid-reducing activity.

In an embodiment, to understand whether hpa could be found in biomass that is suitable for human consumption, the presence of this compound was analysed by LC-ESI-MS in in cyanobacteria from the LEGE cc collection, in Spirulina and Chlorella tabs from commercial sources, and in spinach and cabbage. When compared to *Cyanobium sp.* LEGE 07175 (100%), *Spirulina sp.* produced a higher amount of hpa (120.4%), while much lower values were observed for *Chlorella vulgaris (18.0%),* spinach (14.7%) and cabbage (33.0%), as shown in Figure 3.

Synthesis of hpa, hfa, and other 13²-hydroxy-pheophorbides can be synthesized through the methanolic allomerization of chlorophyll a (Kuronen et al., 1993).

The synthesis of hfa can follow the route presented in scheme 1. Briefly, the acid hydrolysis of chlorophyll a will yield pheophorbide a (scheme 1, i) (You et al., 2011). The conversion of the carboxylic group of pheophorbide an into an acyl chloride can be done through its reaction with 3,3-dichlorocyclopropene (in basic conditions), or with thionyl chloride (scheme 1, ii). The subsequent esterification reaction of the acyl chloride intermediate with 3,7,11-trimethyl-2-dodecene-1-ol (scheme 1, iii), followed by methanolic allomerization will then yield hfa (scheme 1, iv). The same synthetic route can be adapted for the synthesis of other ester derivatives through the reaction of the acyl chloride intermediate with the respective alchools (scheme 1).

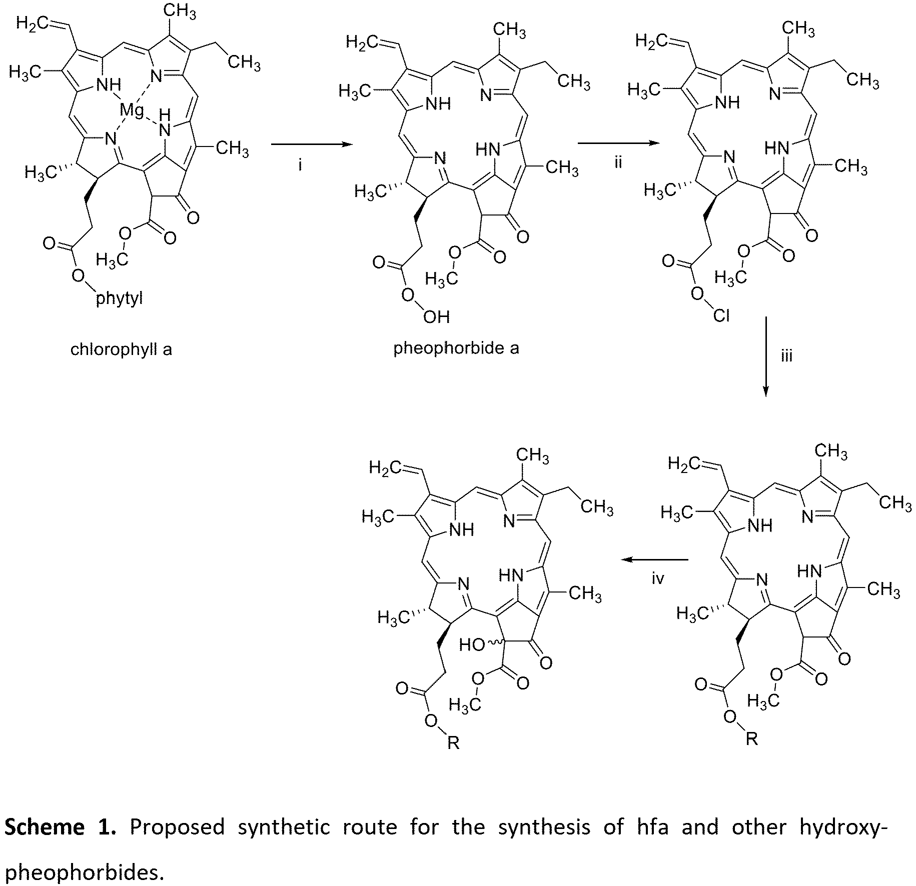

Since chlorophyll derivatives are common to all organisms with chloroplasts (plants, algae, microalgae), it can be expected that organism with a history of food consumption produce these compounds. Those histories of food consumption may lead to the selection of an organism that has the GRAS status (generally regarded as safe). The quantification of *hpa* in extracts of a LEGEcc cyanobacterial strain, *Spirulina* from a commercial source, *Chlorella* from a commercial source and from spinach and cabbage showed that *hpa* can be produced in large quantities in *Spirulina,* which has the GRAS status. Hydroxypheophorbide compounds may be developed as food supplements or nutraceuticals, or as an extract of microalgae rich in those compounds or even as dry biomass with a high content of the compounds - similar to *Spirulina* as nutraceutical with weight reducing properties. The application as concentrated pure compounds in the form of tablets, capsules or candies would be possible as well as the use of the whole dry mass of a plant/algae/microalgae rich in chlorophyll derivatives.

Where singular forms of elements or features are used in the specification of the claims, the plural form is also included, and vice versa, if not specifically excluded. For example, the term "a compound" or "the compound"" also includes the plural forms "compounds" or "the compounds," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

The term "comprising" whenever used in this document is intended to indicate the presence of stated features, integers, steps, components, but not to preclude the presence or addition of one or more other features, integers, steps, components or groups thereof.

### References:

SAKDARAT SET AL, BIOORGANIC & MEDICINAL CHEMISTRY, vol. 17, no. 5, 1 March 2009 (2009-03-01), pages 1857-1860 discloses the anti-herpes simplex activity of the compounds 13-hydroxyphaeophytin a and 13-hydroxyphaeophytin b. ALINE KELY FELÍCIO DE SOUSA SANTOS ET AL, REVISTA BRASILEIRA DE FARMACOGNOSIA- BRAZILIAN JOURNAL OF PHARMACOGNOSY, vol. 25, no. 6, 1 November 2015 (2015-11-01), pages 683-689, discloses the compound 13-hydroxyphaeophytin a for use as antinociceptive NAOYUKI ISHIDA ED - CHANKVETADZE B ET AL, JOURNAL OF CHROMATOGRAPHY A, vol. 1218, no. 34, 22 June 2011 (2011-06-22), pages 5810-5818, discloses 13-hydroxypheophorbide derivatives but no therapeutical activity is ascribed to them. AMIRA ARCINIEGAS ET AL, QUIMICA NOVA, 27 October 2016 (2016-10-27) discloses the compound 13-hydroxyphaeophytin a, but no therapeutical activity is attributed. US 2011/009616 A1 discloses pheothytin derivatives for use in the treatment of arteriosclerosis. SEMAAN D G ET AL, JOURNAL OF ETHNOPHARMACOLOGY, vol. 216, 12 January 2018 (2018-01-12), pages 8-17 discloses pheothytin derivatives for use in the treatment of metabolic disorders such as obesity and diabetes.
- Castro, M.; Preto, M.; Vasconcelos, V.; Urbatzka, R. Obesity: The metabolic disease, advances on drug discovery and natural product research. Curr Top Med Chem 2016, 16, 2577-2604, doi: 10.2174/1568026616666160415155644.
- Cheng, H.H.; Wang, H.K.; Ito, J.; Bastow, K.F.; Tachibana, Y.; Nakanishi, Y.; Xu, Z.; Luo, T.Y.; Lee, K.H. Cytotoxic pheophorbide-related compounds from Clerodendrum calamitosum and C. cyrtophyllum. J Nat Prod 2001, 64, 915-919, doi: 10.1021/np000595b.
- D.G. Semaan, J.O. Igoli, L. Young, A.I. Gray, E.G. Rowan, E. Marrero, In vitro antidiabetic effect of flavonoids and pheophytins from Allophylus cominia Sw. on the glucose uptake assays by HepG2, L6, 3T3-L1 and fat accumulation in 3T3-L1 adipocytes, J Ethnopharmacol, 216, 2018, 8-17, doi.org/10.1016/j.jep.2018.01.014.
- Ferruzzi, M.; Böhm, V.; Courtney, P.; Schwartz, S. Antioxidant and antimutagenic activity of dietary chlorophyll derivatives determined by radical scavenging and bacterial reverse mutagenesis assays. J Food Sci Technol 2002, 67, 2589-2595, doi: 10.1111/j.1365-2621.2002.tb08782.x.
- Gomes, R.A.; Teles, Y.C.F.; Pereira, Fd.O.; Rodrigues, L.Ad.S.; Lima, Ed.O.; Agra Md.F.; Souza, Md.F.Vd. Phytoconstituents from Sidastrum micranthum (A. St.-Hil.) Fryxell (Malvaceae) and antimicrobial activity of pheophytin a. Braz J Pharm Sci 2015, 51, 861-867, doi: 10.1590/S1984-82502015000400012;
- Hayashi, K.-I.; Nozaki, H.; Kamei, Y. Pheophytin A, a low molecular weight compound found in the marine brown alga Sargassum fulvellum, promotes the differentiation of PC12 cells. Int. J. Dev. Neurosci. 2007, 25, 63-68, doi: 10.1016/j.ijdevneu.2006.09.323
- Kang, Y-R.; Park, J.; Jung, S.K.; Chang, Y.H. Synthesis, characterization, and functional properties of chlorophylls, pheophytins, and Zn-pheophytins. Food Chem 2018, 245, 943-950, doi: doi.org/10.1016/j.foodchem.2017.11.079.
- Kuronen, P., Hyvärinen, K., Hynninen, P. H., & Kilpeläinen, I. (1993). High-performance liquid chromatographic separation and isolation of the methanolic allomerization products of chlorophyll a. J Chromatogr A, 654(1), 93-104. doi:10.1016/0021-9673(93)83068-4.
- Ramos, V.; Morais, J.; Castelo-Branco, R.; Pinheiro, A.; Martins, J.; Regueiras, A.; Pereira, A.L.; Lopes, V.R.; Frazão, B.; Gomes, D.; Moreira, C.; Costa, M.S.; Brûle, S.; Faustino, S.; Martins, R.; Saker, M.; Osswald, J.; Leão, P.N.; Vasconcelos, V.M. Cyanobacterial diversity held in microbial biological resource centers as a biotechnological asset: the case study of the newly established LEGE culture collection. J Appl Phycol 2018, 30, 1437-1451, doi: 10.1007/s10811-017-1369-y.
- Subramoniam, A.; Asha, V.V.; Nair, S.A.; Sasidharan, S.P.; Sureshkumar, P.K.; Rajendran, K.N.; Karunagaran, D.; Ramalingam, K. Chlorophyll revisited: Anti-inflammatory activities of chlorophyll a and inhibition of expression of TNF-α gene by the same. Inflammation 2012, 35, 959-966, doi: 10.1007/s10753-011-9399-0.
- Shu, M.H.; Appleton, D.; Zandi, K.; AbuBakar, S. Anti-inflammatory, gastroprotective and antiulcerogenic effects of red algae Gracilaria changii (Gracilariales, Rhodophyta) extract. BMC Complement Altern Med 2013, 13, 61, doi: 10.1186/1472-6882-13-61.
- Turner, P.A.; Harris, L.M.; Purser, C.A.; Baker, R.C.; Janorkar, A.V. A surface-tethered spheroid model for functional evaluation of 3T3-L1 adipocytes. Biotechnol Bioeng 2014, 111, 174-183, doi: 10.1002/bit.25099.
- Urbatzka, R.; Freitas, S.; Palmeira, A.; Almeida, T.; Moreira, J.; Azevedo, C.; Afonso, C.; Correia-da-Silva, M.; Sousa, E.; Pinto, M.; Vasconcelos, V. Lipid reducing activity and toxicity profiles of a library of polyphenol derivatives. Eur J Med Chem 2018, 151, 272-284, doi: 10.1016/j.ejmech.2018.03.036.
- Wang, S-Y.; Tseng, C-P.; Tsai, K-C.; Lin, C-F.; Wen, C-Y.; Tsay, H-S.; Sakamoto, N.; Tseng, C-H.; Cheng, J-C. Bioactivity-guided screening identifies pheophytin a as a potent anti-hepatitis C virus compound from Lonicera hypoglauca Miq. Biochem Biophys Res Commun 2009, 385, 230-235, doi: 10.1016/j.bbrc.2009.05.043
- You, H., Yoon, H-E., Yoon, J-H., Ko, H., & Kim, Y-C. (2011). Synthesis of pheophorbide-a conjugates with anticancer drugs as potential cancer diagnostic and therapeutic agents. BioorgMed Chem, 19(18), 5383-5391. https://doi.org/10.1016/j.bmc.2011.07.058.
- Zhao, Y.; Wang, X.; Wang, H.; Liu, T.; Xin, Z. Two new noroleanane-type triterpene saponins from the methanol extract of Salicornia herbacea. Food Chem 2014, 151, 101-109, doi: 10.1016/j.foodchem.2013.11.030.

## Claims

1. A compound or a pharmaceutically acceptable salt, hydrate, solvate, N-oxide, stereoisomer, diastereoisomer, enantiomer, atropisomer, or polymorph thereof, for use in the therapy or treatment of metabolic diseases of the following formula: 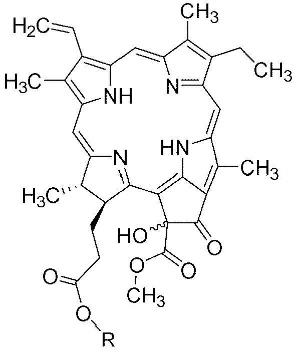 wherein R is CₙH(₂ₙ₋₁) and n is an entire number multiple of 5.

2. The compound for use according to the previous claim wherein n is selected from 5 or 10 or 15 or 20 or 25 or 30, preferably 15 or 20.

3. The compound for use according to any of the previous claims wherein R is C₅H₉, C₁₀H₁₉ or C₁₅H₂₉ or C₂₀H₃₉ or C₂₅H₄₉ or C₃₀H₅₉, preferably C₁₅H₂₉ or C₂₀H₃₉.

4. The compound for use according to any of the previous claims wherein R is 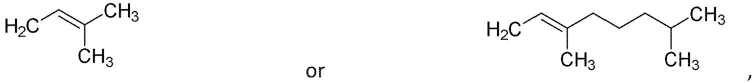 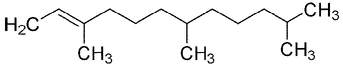 or 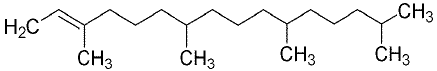 or 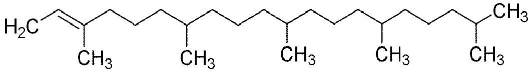 or 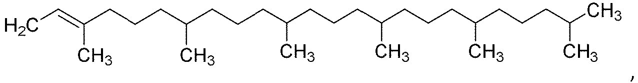 preferably 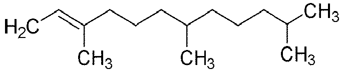 or

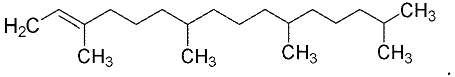

5. The compound for use according to any of the previous claims, wherein the formula is: 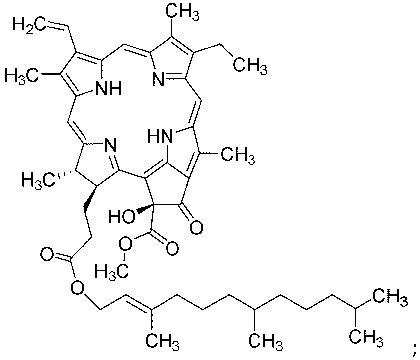 or

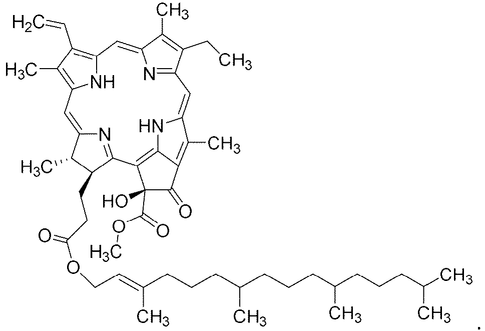

6. The compound according to any of the previous claim for use in the therapy or treatment of obesity, an obesity-related disorder, an obesity related disease, overweight, an obesity-related condition, or lipid obesity disorders; preferably for use in the therapy or treatment of morbid obesity, visceral fat obesity, diabetes, insulin-resistance syndrome, nonalcoholic steatohepatitis, a cardiovascular disorder, renal disorders, pancreatic disorders, polycystic ovary syndrome, metabolic syndrome or fatty liver.

7. A compound or a pharmaceutically acceptable salt, hydrate, solvate, N-oxide, stereoisomer, diastereoisomer, enantiomer, atropisomer, or polymorph thereof of the following formula: 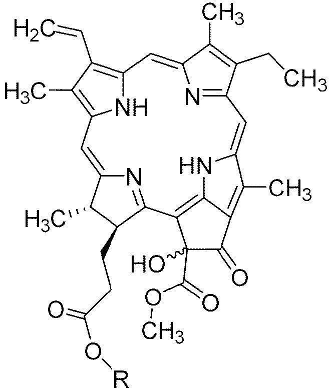
wherein R is CₙH(₂ₙ₋₁)
n is an entire number multiple of 5; wherein n is selected from 5 or 10 or 15 or 25 or 30.

8. The compound according to the previous claim, wherein R is C₅H₉, C₁₀H₁₉ or C₁₅H₂₉ or C₂₅H₄₉ or C₃₀H₅₉, preferably C₁₅H₂₉.

9. The compound according to any of the previous claims 7-8, wherein R is 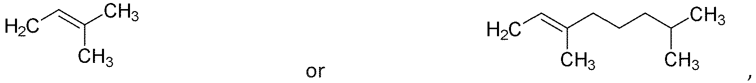 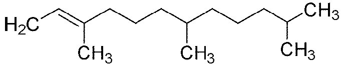 or 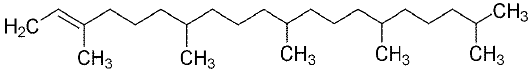 or 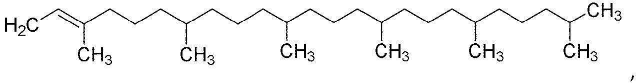 preferably

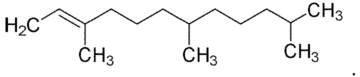

10. The compound according to any of the previous claims 7-9 wherein the formula is:

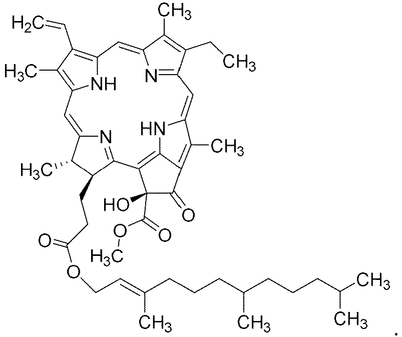

11. A composition for use in the therapy or treatment of metabolic diseases comprising at least a compound, pharmaceutically acceptable salt, hydrate, solvate, N-oxide, stereoisomer, diastereoisomer, enantiomer, atropisomer, or polymorph thereof, according to any of the previous claims and a pharmaceutically acceptable carrier, adjuvant, excipient, emulsification agent, or mixtures thereof.

12. The composition for use according to the previous claim, wherein the composition is a pharmaceutical composition, a foodstuff composition, a dietary composition, a nutraceutical composition or an anti-obesity composition.

13. A composition or a foodstuff/nutraceutical comprising the compound according to any of the previous claims 7-10.

14. Process for the production of the compound described in any of the previous claims, wherein the compound is of formula 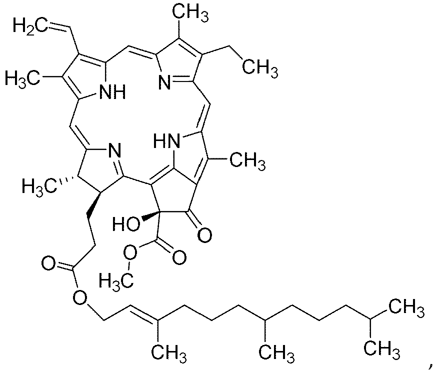 and is isolated from an extract of *Nodosilinea sp.* LEGE06001.

15. Process for the production of the compound described in any of the previous claims, wherein the compound is of formula 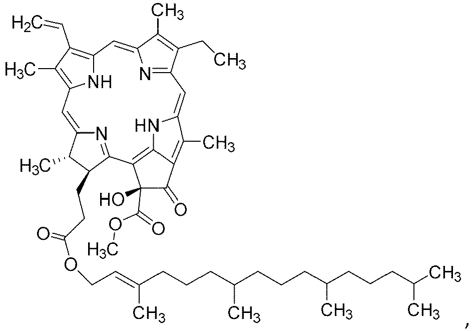 and is isolated from an extract of Cyanobium sp. LEGE 07175.

## Patentansprüche

1. Eine Verbindung oder ein pharmazeutisch akzeptables Salz, Hydrat, Solvat, N-Oxid, Stereoisomer, Diastereoisomer, Enantiomer, Atropisomer oder Polymorph davon zur Verwendung bei der Therapie oder Behandlung von Stoffwechselerkrankungen der folgenden Formel: 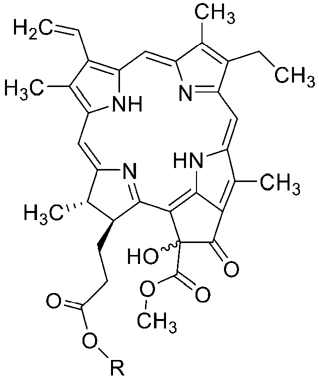 wobei R für CₙH(₂ₙ₋₁) steht und n ein ganzzahliges Vielfaches von 5 ist.

2. Die Verbindung zur Verwendung nach dem vorhergehenden Anspruch, wobei n aus 5 oder 10 oder 15 oder 20 oder 25 oder 30 ausgewählt ist, vorzugsweise 15 oder 20.

3. Die Verbindung zur Verwendung nach einem der vorhergehenden Ansprüche, wobei R C₅H₉, C₁₀H₁₉ oder C₁₅H₂₉ oder C₂₀H₃₉ oder C₂₅H₄₉ oder C₃₀H₅₉ ist, vorzugsweise C₁₅H₂₉ oder C₂₀H₃₉.

4. Die Verbindung zur Verwendung nach einem der vorhergehenden Ansprüche, wobei R 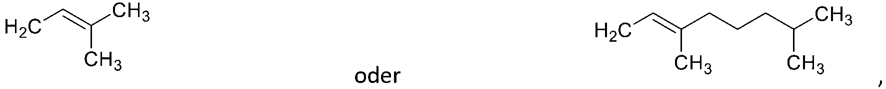 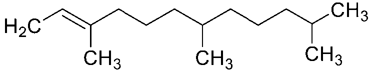 oder 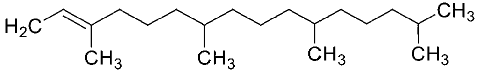 oder 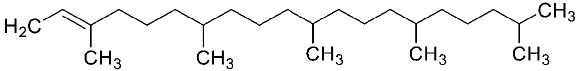 oder 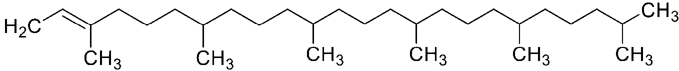 ist, vorzugsweise 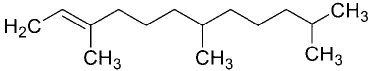 oder

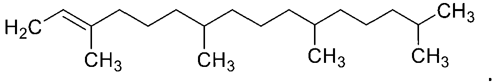

5. Die Verbindung zur Verwendung nach einem der vorhergehenden Ansprüche, wobei die Formel: 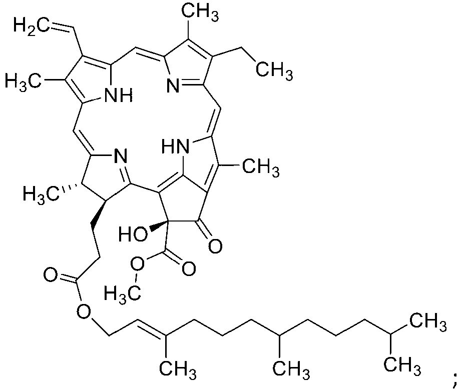 oder 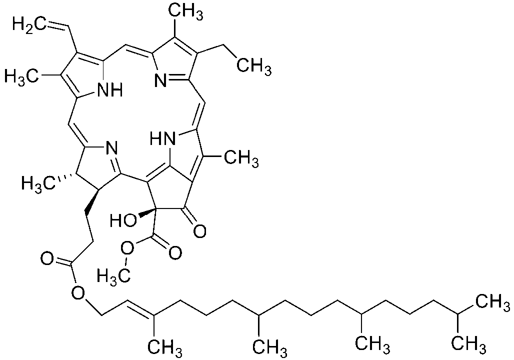 ist.

6. Die Verbindung nach einem der vorhergehenden Ansprüche zur Verwendung bei der Therapie oder Behandlung von Adipositas, einer mit Adipositas verbundenen Störung, einer mit Adipositas verbundenen Erkrankung, Übergewicht, einer mit Adipositas verbundenen Erkrankung oder von Lipid-Adipositas-Erkrankungen; vorzugsweise zur Verwendung bei der Therapie oder Behandlung von morbider Adipositas, viszeraler Fett-Adipositas, Diabetes, Insulinresistenzsyndrom, nichtalkoholischer Steatohepatitis, einer kardiovaskulären Störung, Nierenerkrankungen, Pankreaserkrankungen, polyzystischem Ovarialsyndrom, metabolischem Syndrom oder Fettleber.

7. Eine Verbindung oder ein pharmazeutisch akzeptables Salz, Hydrat, Solvat, N-Oxid, Stereoisomer, Diastereoisomer, Enantiomer, Atropisomer oder Polymorph davon der folgenden Formel: 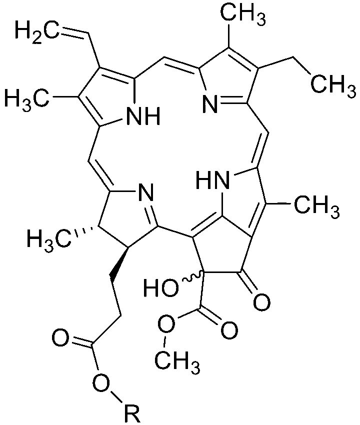
wobei R für CₙH(₂ₙ₋₁) steht
n ein ganzzahliges Vielfaches von 5 ist; wobei n aus 5 oder 10 oder 15 oder 25 oder 30 ausgewählt ist.

8. Die Verbindung nach dem vorhergehenden Anspruch, wobei R C₅H₉, C₁₀H₁₉ oder C₁₅H₂₉ oder C₂₅H₄₉ oder C₃₀H₅₉ ist, vorzugsweise C₁₅H₂₉.

9. Die Verbindung nach einem der vorhergehenden Ansprüche 7-8, wobei R 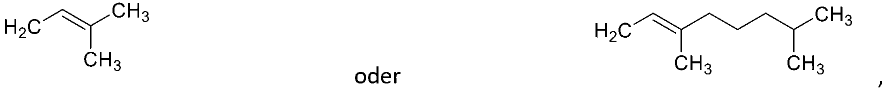 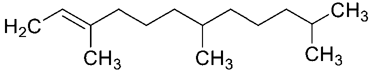 oder 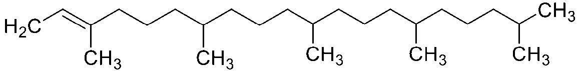 oder 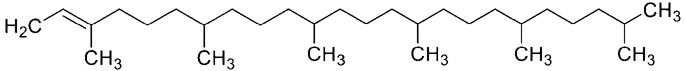 ist, vorzugsweise

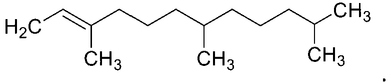

10. Die Verbindung nach einem der vorhergehenden Ansprüche 7-9, wobei die Formel: 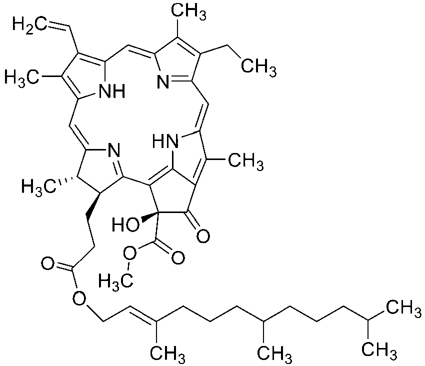 ist.

11. Eine Zusammensetzung zur Verwendung bei der Therapie oder Behandlung von Stoffwechselerkrankungen, umfassend mindestens eine Verbindung, ein pharmazeutisch akzeptables Salz, Hydrat, Solvat, N-Oxid, Stereoisomer, Diastereoisomer, Enantiomer, Atropisomer oder Polymorph davon nach einem der vorhergehenden Ansprüche und einen pharmazeutisch akzeptablen Träger, Adjuvans, Hilfsstoff, Emulgierungsmittel oder Mischungen davon.

12. Die Zusammensetzung zur Verwendung nach dem vorhergehenden Anspruch, wobei die Zusammensetzung eine pharmazeutische Zusammensetzung, eine Lebensmittelzusammensetzung, eine diätetische Zusammensetzung, eine nutraceutische Zusammensetzung oder eine Anti-Adipositas-Zusammensetzung ist.

13. Eine Zusammensetzung oder ein Lebensmittel/Nutraceutikum, umfassend die Verbindung nach einem der vorhergehenden Ansprüche 7-10.

14. Verfahren zur Herstellung der in einem der vorhergehenden Ansprüche beschriebenen Verbindung, wobei die Verbindung die Formel 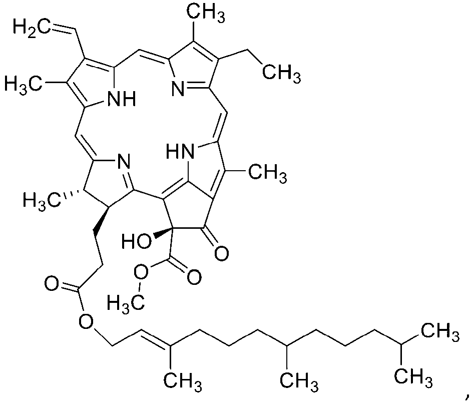 aufweist und aus einem Extrakt von *Nodosilinea sp.* LEGE06001 isoliert wird.

15. Verfahren zur Herstellung der in einem der vorhergehenden Ansprüche beschriebenen Verbindung, wobei die Verbindung die Formel 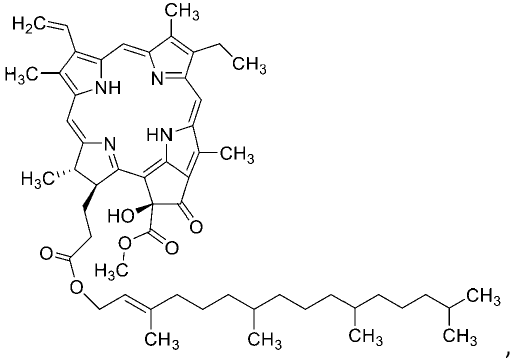 aufweist und aus einem Extrakt von *Cyanobium sp.* LEGE 07175 isoliert wird.

## Revendications

1. Un composé ou un sel, hydrate, solvate, N-oxyde, stéréoisomère, diastéréoisomère, énantiomère, atropisomère ou polymorphe pharmaceutiquement acceptable de celui-ci, destiné à être utilisé dans la thérapie ou le traitement de maladies métaboliques, de la formule suivante : 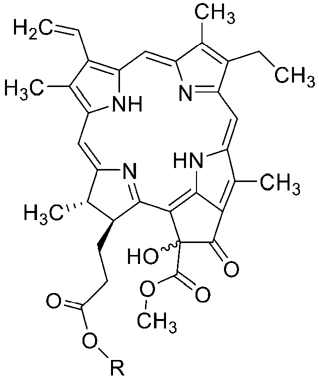 dans laquelle R est CₙH(₂ₙ₋₁) et n est un nombre entier multiple de 5.

2. Le composé destiné à être utilisé selon la revendication précédente, dans lequel n est choisi parmi 5 ou 10 ou 15 ou 20 ou 25 ou 30, de préférence 15 ou 20.

3. Le composé destiné à être utilisé selon l'une quelconque des revendications précédentes, dans lequel R est C₅H₉, C₁₀H₁₉ ou C₁₅H₂₉ ou C₂₀H₃₉ ou C₂₅H₄₉ ou C₃₀H₅₉, de préférence C₁₅H₂₉ ou C₂₀H₃₉.

4. Le composé destiné à être utilisé selon l'une quelconque des revendications précédentes, dans lequel R est 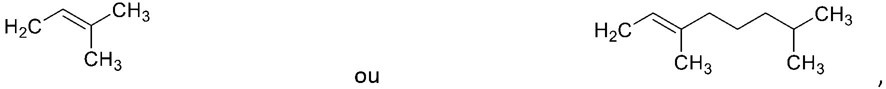 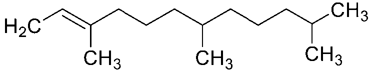 ou 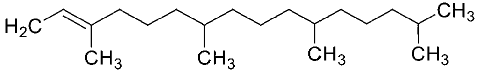 ou 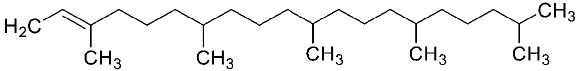 ou 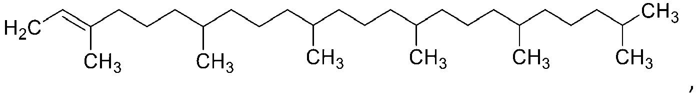 de préférence 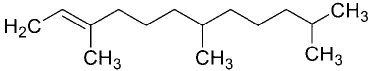 ou

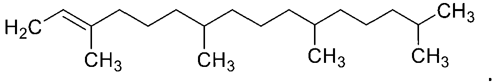

5. Le composé destiné à être utilisé selon l'une quelconque des revendications précédentes, dans lequel la formule est : 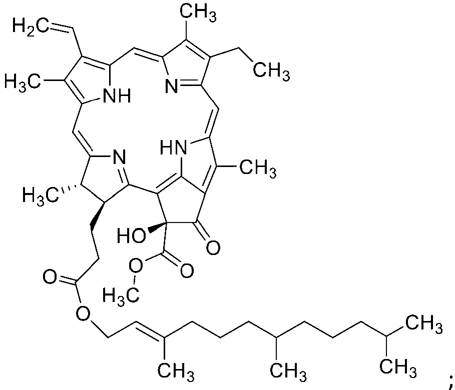 ou

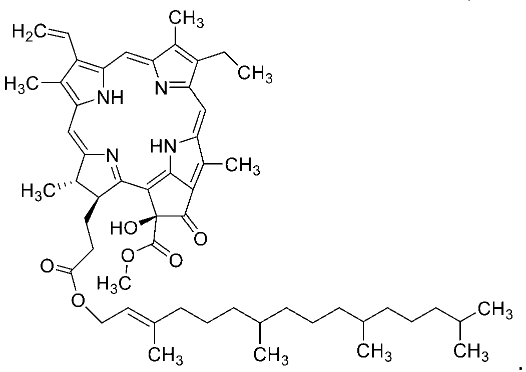

6. Le composé selon l'une quelconque des revendications précédentes, destiné à être utilisé dans la thérapie ou le traitement de l'obésité, d'un trouble lié à l'obésité, d'une maladie liée à l'obésité, du surpoids, d'une affection liée à l'obésité ou de troubles lipidiques liés à l'obésité ; de préférence destiné à être utilisé dans la thérapie ou le traitement de l'obésité morbide, de l'obésité avec graisse viscérale, du diabète, du syndrome de résistance à l'insuline, de la stéatohépatite non alcoolique, d'un trouble cardiovasculaire, de troubles rénaux, de troubles pancréatiques, du syndrome des ovaires polykystiques, du syndrome métabolique ou de la stéatose hépatique.

7. Un composé ou un sel, hydrate, solvate, N-oxyde, stéréoisomère, diastéréoisomère, énantiomère, atropisomère ou polymorphe pharmaceutiquement acceptable de celui-ci, de la formule suivante : 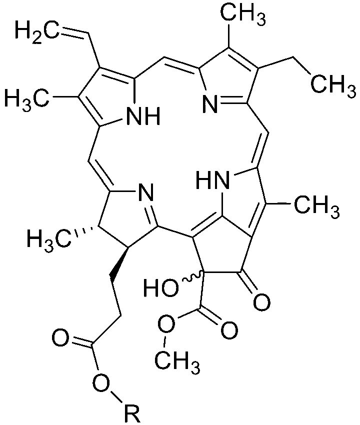
dans laquelle R est CₙH(₂ₙ₋₁)
n est un nombre entier multiple de 5 ; dans laquelle n est choisi parmi 5 ou 10 ou 15 ou 25 ou 30.

8. Le composé selon la revendication précédente, dans lequel R est C₅H₉, C₁₀H₁₉ ou C₁₅H₂₉ ou C₂₅H₄₉ ou C₃₀H₅₉, de préférence C₁₅H₂₉.

9. Le composé selon l'une quelconque des revendications précédentes 7-8, dans lequel R est 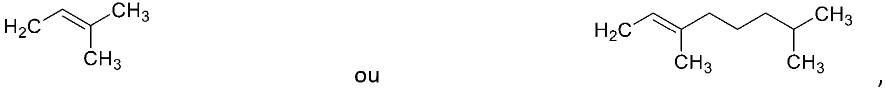 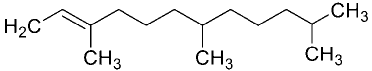 ou 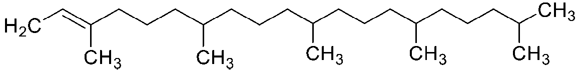 ou 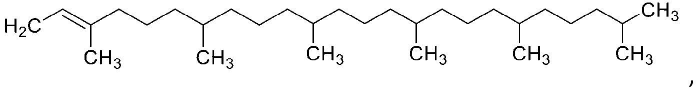 de préférence

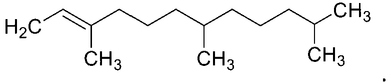

10. Le composé selon l'une quelconque des revendications précédentes 7-9, dans lequel la formule est :

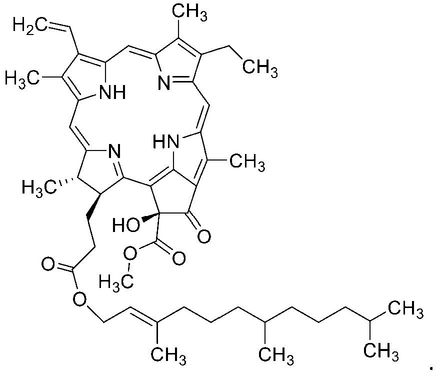

11. Une composition destinée à être utilisée dans la thérapie ou le traitement de maladies métaboliques comprenant au moins un composé, un sel, hydrate, solvate, N-oxyde, stéréoisomère, diastéréoisomère, énantiomère, atropisomère ou polymorphe pharmaceutiquement acceptable de celui-ci, selon l'une quelconque des revendications précédentes, et un support, adjuvant, excipient, agent d'émulsification ou leurs mélanges pharmaceutiquement acceptable.

12. La composition destinée à être utilisée selon la revendication précédente, dans laquelle la composition est une composition pharmaceutique, une composition de denrée alimentaire, une composition diététique, une composition nutraceutique ou une composition anti-obésité.

13. Une composition ou une denrée alimentaire/nutraceutique comprenant le composé selon l'une quelconque des revendications précédentes 7-10.

14. Procédé pour la production du composé décrit dans l'une quelconque des revendications précédentes, dans lequel le composé est de formule 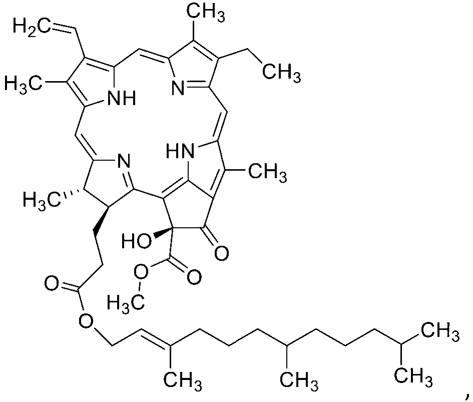 et est isolé à partir d'un extrait de *Nodosilinea sp.* LEGE06001.

15. Procédé pour la production du composé décrit dans l'une quelconque des revendications précédentes, dans lequel le composé est de formule 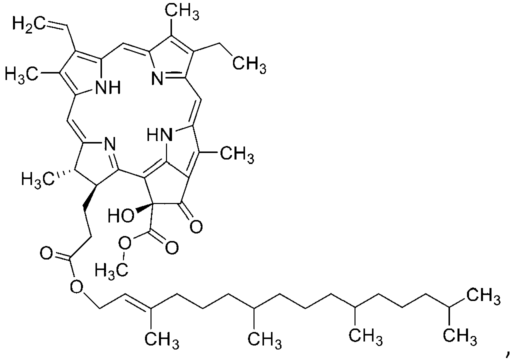 et est isolé à partir d'un extrait de *Cyanobium sp.* LEGE 07175.
